# EUROPÄISCHE PATENTSCHRIFT

(11) **EP 3 337 410 B1**
(45) Veröffentlichungstag und Bekanntmachung des Hinweises auf die Patenterteilung: **17.03.2021**
(21) Anmeldenummer: 16748291.8
(22) Anmeldetag: 02.08.2016
(51) Int. Cl.: A61B 17/135, A61B 17/132, A61M 1/36, A61M 1/14

(54) **MEDIZINTECHNISCHE BEHANDLUNGSEINHEIT MIT EINER VORRICHTUNG ZUR BLUTSTILLUNG AN DEN EINSTICHSTELLEN DER GEFÄSSE VON PATIENTEN UND EINER BLUTBEHANDLUNGSVORRICHTUNG**
MEDICAL TREATMENT UNIT WITH A DEVICE FOR STOPPING BLEEDING AT THE PUNCTURE SITES OF THE VESSELS OF PATIENTS AND A BLOOD TREATMENT DEVICE
UNITÉ DE TRAITEMENT MÉDICAL AVEC UN DISPOSITIF D'HÉMOSTASE AUX POINTS DE PIQÛRE DES VAISSEAUX DE PATIENTS ET UN DISPOSITIF DE TRAITEMENT DU SANG

(30) Priorität: 17.08.2015 DE 102015010743
(43) Veröffentlichungstag der Anmeldung: 27.06.2018
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE); KOPPERSCHMIDT, Pascal, 97456 Dittelbrunn (DE)
(86) Internationale Anmeldenummer: PCT/EP2016/068431
(87) Internationale Veröffentlichungsnummer: WO 2017/029111

(56) Entgegenhaltungen:
- EP-A2- 1 382 306
- WO-A1-98/46144
- CN-A- 104 337 559
- CN-U- 202 235 549
- US-A1- 2003 236 548
- US-A1- 2007 032 818
- US-A1- 2013 085 524
- US-A1- 2015 018 869
- US-A1- 2015 201 948

## Beschreibung

Vorrichtung und Verfahren zur Blutstillung an den Einstichstellen der Gefäße von Patienten, sowie Auswerteeinheit mit Sensor
Die Erfindung bezieht sich auf eine medizinische Behandlungseinheit mit einer Vorrichtung zum Stillen von Blutungen an mindestens einer Einstichstelle eines Gefäßes eines Patienten, der einer extrakorporalen Behandlung, insbesondere einer Dialyse unterzogen wurde, und mit einer extrakorporalen Blutbehandlungsvorrichtung, insbesondere einer Hämodialysevorrichtung.

Es ist seit langem bekannt, zur Kontrolle von Blutungen bis hin zur vollständigen Stillung derselben Vorrichtungen einzusetzen, die der Kompression von Gefäßen dienen, wodurch der Blutfluss in diesen reduziert oder vollständig unterbrochen werden kann. Beispielsweise sind Kompressionsriemen zur Kontrolle von Blutungen sowohl aus den Überlieferungen griechischer Gelehrter als auch aus dem Römischen Reich bekannt. Militärchirurgen verwendeten diese, um Blutungen bei Amputationen zu kontrollieren. Zur Versorgung sehr starker Blutungen entwickelte der französische Chirurg Louis Petit im Jahr 1718 ein mechanisches Instrument mit einer Spannschraube, das er Tourniquet nannte, Ein Stauschlauch aus flexiblem Material geht auf eine Erfindung von Johann von Esmarch aus dem Jahr 1873 zurück mit dem Vorteil, dass sich hierbei keine mechanischen Teile lösen konnten.

Die modernen Tourniquets werden entweder pneumatisch oder mechanisch betrieben und ähneln in Aussehen und Funktion einer Blutdruckmanschette. Mechanische Tourniquets funktionieren durch Verkürzung eines Bandes durch Rotation. Dabei kann es jedoch zu Verletzungen des Gewebes und der Nerven kommen. Ein weiteres Risiko besteht in der Bildung von Blutgerinnseln. Um den notwendigen Druck zu erreichen, wird bei pneumatischen Tourniquets Luft in einen Ballon gepumpt, der in eine Manschette eingearbeitet ist. Diese Verfahrensweise birgt geringere Verletzungsgefahren.

Vorrichtungen zur Kompression von Blutgefäßen werden in der Medizin zu verschiedenen Zwecken eingesetzt, beispielsweise zur Stauung der oberflächlichen Venen bei der Blutentnahme, bei der Phlebographie oder in der Chirurgie von Extremitäten, um in einem blutarmen Gebiet operieren zu können. Auch bei der Anwendung von extrakorporalen Verfahren, bei welchen dem Patienten während der Behandlung Blut mittels einer Hohlnadel durch die Haut aus einem Gefäß entnommen, in einen extrakorporalen Kreislauf geleitet und nach der Behandlung wieder zugeführt wird, werden Vorrichtungen dieser Art verwendet, um nach Beendigung der Therapie und Entfernung der Hohlnadel aus den Gefäßen die Blutung an der Einstichstelle zu stillen.

Um bei der Behandlung mit einem extrakorporalen Verfahren einen ausreichenden Blutfluss zu gewährleisten, werden zur Öffnung der Blutgefäße Hohlnadeln größeren Durchmessers verwendet, so dass nach dem Entfernen der Nadel zum Wiederverschließen der Einstichstelle ein erheblicher dauerhafter Druck erforderlich ist. Ferner werden bei extrakorporalen Verfahren häufig antikoagulierende Mittel, wie beispielsweise Heparin eingesetzt, so dass die Nachblutungszeit verlängert ist.

Wie einfach bzw. in welcher Zeit sich die Blutung an den Einstichstellen nach Entfernung der Hohlnadeln stillen lässt, ist von Patient zu Patient sehr unterschiedlich und von verschiedenen Faktoren abhängig, wie beispielsweise der Beschaffenheit der Gefäßwand, der Blutviskosität, dem Blutdruck oder dem Alter des Patienten. Außerdem ist es von Belang, ob es sich bei dem punktierten Gefäß um eine Arterie oder eine Vene handelt, da arterielle Blutungen schwieriger zu stillen sind. Auch die Punktionsart (z.B. Strickleiterpunktion, Knopfloch-Punktion, Area-Punktion, etc.) und das Verhalten des Patienten nach Entfernung der Hohlnadel nehmen erheblich Einfluss auf die Nachblutungszeit. Diese ist beispielsweise verlängert, wenn der Patient sich stark bewegt und dadurch den Blutdruck und den Druck auf die Einstichstelle erhöht.

Daher kommt es regemäßig dazu, dass ein Behandlungsplatz nicht frei wird, weil die Blutung des Patienten noch nicht gestillt werden konnte.

Durch die verlängerte Nachblutungszeit können auch hygienische Probleme entstehen.

Erfolgt das Anbringen eines dauerhaften Drucks auf die Einstichstellen des Zugangs durch das Personal, ergibt sich außerdem ein hoher Zeit- und Kostenaufwand.

Wird das Abdrücken dem Patienten selbst überlassen, ist dies für ihn sehr unkomfortabel und es kann zu einem unzureichenden Gefäßverschluss und damit zu einer erneuten Blutung kommen. Hat der Patient den Behandlungsbereich bereits verlassen, kann dies zu gesundheitlichen Problemen beim Patienten und zu organisatorischen Schwierigkeiten in der Behandlungseinheit führen.

Beim Abdrücken ist es außerdem schwierig, dauerhaft eine konstante Druckkraft aufrecht zu erhalten. Ferner ist es schwierig, einen Druck aufzuwenden, der dem zur Vermeidung einer Nachblutung angepassten Druckbedarf Rechnung trägt.

Daher wurden bereits zahlreiche Vorrichtungen entwickelt, die das Abdrücken eines Gefäßes auf mechanische Art und Weise gewährleisten.

So beschreibt das Dokument EP 0 462 088 eine Vorrichtung zum Abdrücken der Oberschenkelarterie, die aus einem Gürtel und einer Grundplatte mit einem unterdrucksetzenden Mittel besteht, wobei die Grundplatte und das unterdrucksetzenden Mittel zumindest teilweise aus durchsichtigem Material hergestellt sind.

Die EP 1 382 306 schlägt eine blutstillende Vorrichtung vor, die ein flexibles Band mit einer zur Innenseite gekrümmten Platte umfasst. Letztere weist einen Ballon auf, in den ein Fluid zur Erweiterung eingeleitet werden kann und ein kleineres Anpresselement, das mit einem zweiten Fluid gefüllt, gegen den diesen Ballon drückt.

Aus der EP 1 125 554 ist ein Venenstaugerät mit einer Druckschlauchmanschette, einer Druckquelle und einem Druckeinstell-Hilfsmittel bekannt. Die Druckkraft wird so eingestellt, dass ein Abdrücken einer Vene bewirkt wird, der Strömungsquerschnitt einer Arterie jedoch zumindest teilweise erhalten bleibt. Der Druckwert wird entweder vorab durch Versuche für unterschiedliche Patienten bestimmt oder über eine Blutdruckmessung individuell berechnet.

Die DE 298 15 375 bezieht sich auf ein Abdrückband zum Verschließen der Einstichstellen von Gefäßen von Dialysepatienten. Das Band besitzt ein der Einstichstelle zugekehrtes elastisches Material mit einem Stopfen, der der Größe der Einstichstelle angepasst ist und druckbelastend auf diese wirkt.

Die WO 2011/090429 beschreibt eine Kompressionseinheit mit einem aufblasbaren Ballon zum Abdrücken einer Punktionsstelle an einer Arterie eines Patienten

Das Dokument US2013085524 A1 beschreibt ein femorales Kompressionssystem zur Anwendung von Kompression gegen eine Punktionsstelle eines Gefässes bei einem Patienten und ein Verfahren zur Anwendung von Kompression mit einem femoralen Kompressionssystem. Das Kompressionssystem umfasst ein aufblasbares Kompressionselement, das geeignet ist, einen Druck gegen die Punktionsstelle auszuüben, eine Spanneinheit, die geeignet ist, sich um einen Teil oder den ganzen Körper des Patienten zu erstrecken, um das Kompressionselement an der Punktionsstelle zu fixieren und zu spannen, eine Pumpe, die geeignet ist, das Kompressionselement aufzublasen, ein Ventil, das geeignet ist, das Kompressionselement zu entlüften, einen Druckwandler, der geeignet ist, den Druck innerhalb des Kompressionselements zu erfassen. Das System umfasst ferner einen Blutdruckimpulsdetektor, der so ausgelegt ist, dass er den Blutdruckimpuls des Patienten erfasst und in Abhängigkeit davon ein Impulssignal erzeugt, das an eine Steuereinheit angelegt wird, die mit der Pumpe, dem Ventil und dem Druckwandler verbunden ist, wobei die Steuereinheit so ausgelegt ist, dass sie den Druck innerhalb des Kompressionselements in Abhängigkeit von dem Impulssignal steuert, indem sie Steuersignale an die Pumpe und das Ventil anlegt.

US2015201948 A1 und US2003236548 A1 beschreiben weitere Kompressionssysteme mit Sensoren und Kontrolleinheiten.

Die Hämodialyse ist das gebräuchlichste extrakorporale Verfahren. Hierbei wird entweder über dieselbe Hohlnadel oder eine zweite, die ebenfalls in ein blutführendes Gefäß eingeführt ist, Blut entnommen und wieder zurückgeführt. Trotz des Bedarfs gibt es bisher keine geeigneten blutstillenden Mittel, die insbesondere für die Anwendung bei Dialysepatienten einsetzbar sind. Ein zusätzliches Problem, das bei Dialysepatienten auftritt, ist, dass die Einstichstelle regelmäßig in kurzen Abständen punktiert werden muss. Um sie nicht übermäßig zu belasten, wird in der Regel operativ ein Zugang in Form einer Gefäßgestaltung, beispielsweise einer Fistel oder eines Shunts, gelegt. Fistel und Shunt sind Bereiche mit hohem Blutfluss, so dass beim Stillen der Nachblutung ein hoher Druck aufgewendet werden muss. Ein vollständiges Sperren des Blutflusses sollte jedoch vermieden werden, was problematisch durchzuführen ist. Es wird sogar explizit von einigen Gesundheitsbehörden von der Verwendung solcher Manschetten abgeraten, da sie mit einem erhöhten gesundheitlichen Risiko für den Patienten behaftet sind. Beispielhaft wird hier auf die BC Renal Agency "Vascular Access Guidelines" vom 12. Oktober 2011 verwiesen, wo es heißt "Clotting device such as a tourniquet or strap (not recommended)". Daher wird die Blutung bei Dialysepatienten meist nach wie vor durch manuelles Aufdrücken eines Textilstücks, z.B. Gaze, auf die Einstichstellen gestillt.

Der Erfindung liegt daher die Aufgabe zugrunde, eine medizintechnische Behandlungseinheit zur Verfügung zu stellen, die es ermöglicht, die Blutung an den Einstichstellen der Gefäße von Patienten, die einer extrakorporalen Behandlung und insbesondere der Dialyse unterzogen wurden, nachhaltig sicher und in kürzest möglicher Zeit zu stillen und somit die Nachteile der bisherigen Systeme und Vorgehensweise auszuräumen.

Eine weitere Aufgabe der Erfindung ist die leichte Anwendbarkeit, d.h. das komfortable, leichte Anlegen der Vorrichtung.

Darüber hinaus soll die Vorrichtung kostengünstig und einfach herstellbar sein.

Die Aufgabe wird erfindungsgemäß durch die Merkmale des Anspruchs 1 gelöst.

Anspruch 1 betrifft eine medizintechnische Behandlungseinheit mit einer Vorrichtung und einer Blutbehandlungsvorrichtung, insbesondere einer Hämodialysevorrichtung, die eingerichtet ist, mit der Datenübertragungseinrichtung zu kommunizieren.

Die erfindungsgemäße Vorrichtung weist einen bandförmigen Grundkörper mit Verschlussmittel und wenigstens eine erste expandierbare Kammer an dem bandförmigen Grundkörper auf.

Der bandförmige Grundkörper besteht vorzugsweise aus leicht zu reinigendem und vorzugsweise flexiblem oder elastischem Material, wie Stoff, Gummi, Kunststoff oder dergleichen. Kunststoff ist beispielsweise ein relativ gut zu verarbeitender und preiswerter Werkstoff, der es auch erlaubt, den Grundkörper als Einmalartikel zu verwenden.

Der bandförmige Grundkörper kann ein Verschlussmittel aufweisen, durch das er vorzugsweise zu einem Ring oder einem Teilring formbar und durch Umgreifen einer Gliedmaße eines Patienten befestigbar ist. Das Verschlussmittel kann aus Kunststoff bestehen. Als Verschlussmittel kommen beispielsweise nicht kontinuierlich positionierbare Mittel wie Schnallen, Druckknöpfe oder dergleichen in Betracht. Dadurch ist eine bessere Reproduzierbarkeit ohne die Notwendigkeit der Regelung des Füllungsgrads in der oder den Kammern möglich. Aber auch kontinuierlich verstellbare Verschlussmittel, wie beispielsweise Klett- oder Ratschenverschlüsse sind denkbar.

In der ringförmigen Anordnung kann der Grundkörper zumindest in einem Teil des Bereichs der wenigstens ersten Kammer auf einer Außenseite steifer ausgebildet sein als auf einer Innenseite. Durch diese versteiften Bereiche kann die Ausdehnung des Kammerbereichs nach innen stärker als nach außen gerichtet sein.

Für die korrekte Positionierung der Kammer über der Einstichstelle ist die wenigstens erste Kammer zumindest teilweise transparent.

Die Kammer besteht aus flexiblem, vorzugsweise elastischem Material, so dass sie sich bei der Zufuhr des Fluids ausdehnen kann, also expandierbar ist. Da das Volumen der Kammer veränderbar ist, kann die Kraft, mit der auf die Einstichstelle gedrückt wird, durch die Wahl eines geeigneten Drucks eingestellt werden.

Die Abmessungen der Kammer in Längsrichtung sind geringer als die Abmessung des Grundkörpers, vorzugsweise können sie weniger als die Hälfte der Abmessungen des Grundkörpers betragen.

Die Kammer ist über eine Fluidzufuhreinrichtung, die beispielsweise ein Schlauch sein kann, mit einer Fördereinheit, welche beispielsweise eine Pumpe, eine unter Druck stehende Kartusche oder eine Spritze sein kann, zum Einführen eines Fluids in die Kammer verbindbar.

Die Kammer kann auch durch zwei miteinander verbundene flächige Bereiche unterschiedlicher Größe gebildet werden. Dadurch kann das Volumen der Kammer beim Einführen von Fluid vergrößert werden. Die beiden flächigen Bereiche, die die erste Kammer bilden, können unterschiedlich steif sein. Dadurch kann eine Ausdehnung beim Aufblasen gezielt in eine Richtung erfolgen.

Als Fluid wird vorzugsweise Gas verwendet, die Verwendung von Flüssigkeit ist jedoch ebenfalls denkbar.

Zum Ablassen des Fluids weist die Vorrichtung ferner ein Ventil auf, das derart mit der Fluidzuführvorrichtung verbunden ist, dass in der wenigstens ersten Kammer befindliches Fluid ablassbar ist. Als Ventil kann auch die Fördereinheit fungieren, indem sie aktiv Fluid aus der Kammer herausfördert.

Die Vorrichtung weist außerdem eine Steuerung auf. Diese steuert die Fördereinheit und/oder das Ventil und ist mit einem Speicher versehen. In dem Speicher ist wenigstens ein Profil gespeichert, gemäß dem das Fluid in einer definierten Art und Weise in die wenigstens erste Kammer eingeführt oder aus dieser abgelassen werden kann.

Das Profil, mit dem Fluid in die Kammer eingeführt oder aus dieser abgelassen wird, kann durch einen vorgegebenen Maximaldruck charakterisiert sein. Das Profil kann durch verschiedene zeitliche Verläufe, in welchen ein Ventil geöffnet wird und/oder der Öffnungsgrad eines Ventils geändert oder die Fördereinheit betrieben wird, gekennzeichnet sein oder einen linearen, stufenförmigen oder exponentiellen Verlauf aufweisen. Ferner ist vorstellbar, dass das Profil einer bestimmten Messmimik folgt, welche den Blutungsgrad analysiert.

Das Profil kann auch dadurch gekennzeichnet sein, dass der Füllungsgrad in der ersten Kammer zwischenzeitlich erhöht wird, wenn die Fördereinheit gemäß dem Profil angesteuert wird.

In einem Speicher kann eine Mehrzahl von Profilen gespeichert sein, beispielweise wenigstens zwei Profile, die sich darin unterscheiden, dass der Füllungsgrad unterschiedlich schnell absenkbar ist.

Eine Speicherung von Profilen bietet sich insbesondere dann an, wenn derselbe Patient häufiger die Einrichtung zur extrakorporalen Behandlung aufsucht, wie beispielsweise in der Dialyse, da dann verschiedene Profilparameter bekannt sind und auch Behandlungsbesonderheiten berücksichtigt werden können.

Die erfindungsgemäße Vorrichtung kann eine Eingabevorrichtung aufweisen, mit der das wenigstens eine Profil anwählbar ist. Die Eingabevorrichtung kann Bedienelemente zur Auswahl des Profils beinhalten. Ferner kann sie auch eine Anzeige zur Anwahl des Profils aufweisen. Vorzugweise sind die Steuerung und die Eingabevorrichtung in ein gemeinsames, tragbares Gehäuse integriert.

Die erfindungsgemäße Vorrichtung kann auch mindestens einen Sensor zum Messen des Füllungsgrads in der wenigstens ersten Kammer aufweisen und die Fördereinheit und/oder das Ventil können durch die Steuerung auf der Grundlage des vom Sensor übertragenen Messwerts ansteuerbar sein. Dies kann mittels eines in der Steuerung angeordneten Prozessors erreicht werden. Durch Messen eines Istwertes des Füllungsgrades der ersten Kammer kann dieser durch die Steuerung beeinflusst werden. Der Sensor kann an der ersten Kammer angeordnet sein oder in die Zuführeinrichtung oder die Steuerung integriert sein.

Wird als Fluid Gas verwendet, kann der Sensor ein Drucksensor und der Messwert ein Druckwert sein. Bei Hämodialysepatienten sind keine größeren Druckwerte erforderlich, beispielsweise können Drücke zwischen 6666 Pascal (50mmHg) und 19999 Pascal (150mmHg) ausreichend sein, so dass eine kleine Dimensionierung der Fördereinheit möglich ist

Die erfindungsgemäße Vorrichtung ist nicht auf den eigentlichen, am Patienten anzubringenden Grundkörper beschränkt, sondern kann auch andere Elemente wie beispielsweise die Steuerung, die Fluidzuführeinrichtung oder die Fördereinheit, die mit dem Grundkörper verbunden sind, umfassen.

Die Steuerung kann eine handhaltbare Vorrichtung sein, so dass der Patient eine erhöhte Mobilität genießt und beispielsweise die Behandlungseinrichtung verlassen kann. Während der Grundkörper als Einmalartikel ausgestaltet sein kann, kann die Steuerung wiederverwendbar sein. Dazu können die Steuerung und der Grundkörper auch trennbar ausgebildet sein.

In die handhaltbare Vorrichtung können wenigstens eines oder mehrere der Bauteile aus der Gruppe der Bauteile bestehend aus Sensor, Fördereinrichtung oder Eingabevorrichtung integriert sein.

Es ist weiterhin vorstellbar, dass die erfindungsgemäße Vorrichtung eine zweite expandierbare Kammer in dem Grundkörper aufweist, wodurch eine Positionierung der Manschette auch bei zwei Einstichstellen (Entnahmestelle und Rückgabestelle für das Blut) möglich ist und der Druck auf zwei Einstichstellen optimiert angewendet werden kann. Bei Vorhandensein lediglich einer großen Kammer kann der Druck auf den Bereich zwischen den beiden Einstichstellen am größten sein.

Die zweite Kammer kann gleich der ersten Kammer ausgebildet sein. Sowohl die erste Kammer als auch die zweite Kammer können zumindest teilweise oder vollständig aus transparentem Material gebildet werden.

Die beiden Kammern können auf einer Achse senkrecht zu dem bandförmigen Grundkörper angeordnet sein. Diese Anordnung entspricht der relativen Orientierung der beiden Einstichstellen bei einem Dialysepatienten.

Zwischen der ersten Kammer und der zweiten Kammer kann ein gasdurchlässiger Kanal vorgesehen sein. Im Bereich der Kammern kann der Grundkörper eine variierende Steifigkeit aufweisen, wodurch der Kanal beim Füllen der ersten und der zweiten Kammer ausbildbar ist. Der Kanal kann eine verringerte Breite und/oder eine verringerte Höhe im Vergleich zu der ersten Kammer und der zweiten Kammer aufweisen. Der Kanal ermöglicht das Füllen und Entleeren der ersten Kammer und der zweiten Kammer durch lediglich eine Zuführeinrichtung. Eine der Kammern besitzt dabei mindestens eine Zuführ- und eine Ablassstelle für ein Fluid.

Die erste Kammer und die zweite Kammer können auch isoliert voneinander angeordnet sein und jeweils eine eigene Zuführ- und/oder Ablassstelle für ein Fluid besitzen.

Die erfindungsgemäße Vorrichtung kann auch eine zweite Fördereinheit aufweisen, die derart angeordnet ist, dass durch diese ein Fluid aus der zweiten Kammer durch die Zuführeinrichtung zuführbar oder ablassbar ist.

In dem Speicher kann wenigstens ein Profil zum gesteuerten Ablassen des Mediums aus der zweiten Kammer gespeichert sein. Das Profil zum Ablassen von Gas aus der ersten Kammer kann von dem Profil zum Ablassen von Gas aus der zweiten Kammer verschieden sein.

Die Steuerung weist eine Datenübertragungseinrichtung und einen Prozessor auf, wobei der Prozessor geeignet ist, mittels der durch die Datenübertragungseinrichtung übertragenen Daten ein Profil zu bestimmen. Die Eignung kann beispielsweise dadurch erreicht werden, dass es sich bei der Datenübertragungseinrichtung um eine Schnittstelle aus der der Gruppe bestehend aus einer manuellen Eingabevorrichtung, einer drahtlosen oder eine drahtgebundene Kommunikationsschnittstelle handelt. Bei den Daten kann es sich um Daten aus der Gruppe bestehend aus Patientenidentifikation, Blutviskosität, Hämatokrit, Fistel- bzw. Shuntdruck, profilspezifische Kennzahl, Dialysebehandlungsparameter, ACT (activated clotting time") oder Antikoagulationsregimeparameter handeln.

Blutviskosität und Hämatokrit des Blutes kann mittels einer Ultraschall- oder optischen Messeinrichtung, wie sie in Hämodialysegeräten eingesetzt werden, während einer Dialysebehandlung bestimmbar sein.

Der Speicher kann Profile für verschiedene Patienten oder Patientengruppen speichern. Durch Übertragung einer Patientenidentifikation oder einer profilspezifischen Kennzahl kann die Steuerung programmiert sein, ein entsprechendes Profil auszuwählen. Es können blutspezifische Werte zur Auswahl des Profils durch die Steuerung übertragbar sein. Ein blutspezifischer Wert kann beispielsweise die Blutviskosität oder der Hämatokrit sein.

Wenn in dieser Beschreibung ein Profil benannt ist, so kann es sich um das Profil für die erste Kammer und/oder für die zweite Kammer handeln.

Der Prozessor in der Steuervorrichtung kann auch mittels eines im Speicher gespeicherten Programms Instruktionen ausführen, so dass der Prozessor die Fördereinrichtung und/oder das Ventil entsprechend des Profils ansteuert.

Es ist denkbar, dass der Füllungsgrad der Kammer von zumindest einem Sensor gemessen wird und der Messwert durch eine Auswerteeinheit zur Korrektur der Profile erfasst wird. Hierdurch kann der minimale Druck, der zur Stillung der Blutung gerade noch ausreichend ist, gefunden werden.

Der Sensor kann aber auch zur Messung verschiedener anderer Werte, wie beispielsweise Temperatur, Feuchtigkeit usw. dienen und so eine unzureichende Blutstillung identifizieren. Auf der Basis der Messwerte kann ein gesondertes Profil für jede individuelle Situation erstellbar sein.

Die erfindungsgemäße Vorrichtung kann ein System zum Stillen von Blutungen an einer Einstichstelle eines Hämodialysepatienten integriert sein, wobei die Vorrichtung eingerichtet ist, mit einer Blutbehandlungsvorrichtung, insbesondere einer Hämodialysevorrichtung, über die Datenübertragungseinrichtung zu kommunizieren. Dazu kann die Blutbehandlungsvorrichtung ebenfalls eine Datenübertragungseinrichtung aufweisen. Die Datenübertragungseinrichtung kann beispielsweise eine LAN, eine WLAN, eine WiFi, eine Bluetooth oder eine RFID Schnittstelle sein.

Im Folgenden werden Ausführungsbeispiele unter Bezugnahme auf die Zeichnungen näher erläutert.

Es zeigen:
Fig.1a: eine Ausführungsform der erfindungsgemäßen Vorrichtung mit einer Kammer in Aufsicht
Fig.1b: eine Ausführungsform der erfindungsgemäßen Vorrichtung mit einer gefüllten Kammer im Längsschnitt
Fig.2: eine weitere Ausführungsform der erfindungsgemäßen Vorrichtung mit zwei Kammern, die durch einen Kanal 202 miteinander verbunden sind, in Aufsicht
Fig.3: eine weitere Ausführungsform der erfindungsgemäßen Vorrichtung mit zwei voneinander isolierten Kammern in Aufsicht
Fig.4: eine medizintechnische Behandlungseinheit mit der erfindungsgemäßen Vorrichtung und einer extrakorporalen Blutbehandlungsvorrichtung,
Fig.5: Ausführungsformen für Profile zum Ablassen von Fluid aus wenigstens einer der Kammern
Fig.6: eine Ausführungsform der Steuerung mit Anzeige und Bedienelementen
Fig. 7: eine Ausführungsform der Steuerung gemäß Fig. 6 mit einzelnen Bauteilen
Fig. 8: ein Verfahren zum Stillen von Blutungen unter Verwendung der erfindungsgemäßen Vorrichtung

In den Figuren sind entsprechende Merkmale mit denselben Referenznummern versehen.

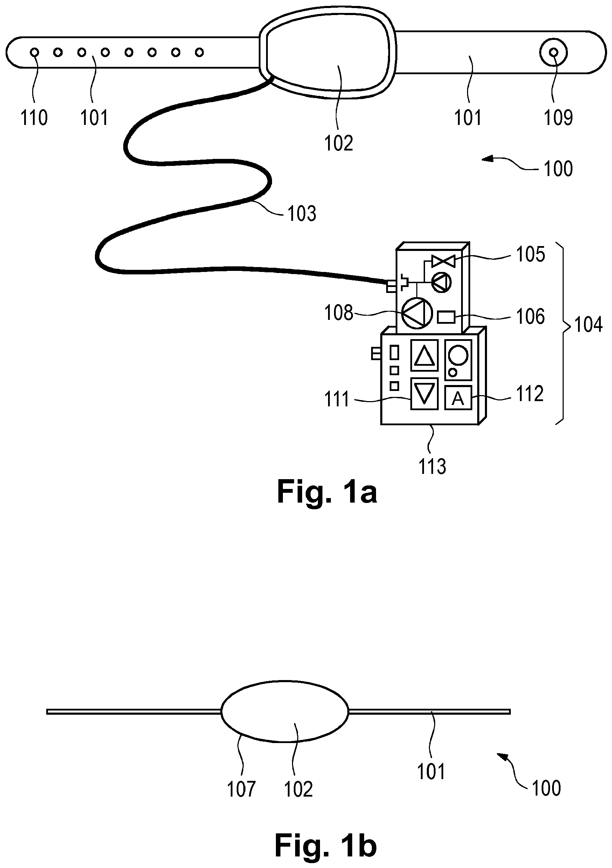

Fig. 1a zeigt die erfindungsgemäße Vorrichtung 100 in inaktivem Zustand. Die Vorrichtung 100 weist einen bandförmigen Grundkörper 101 mit einer expandierbaren Kammer 102 auf, sowie Verbindungsmittel, hier beispielsweise einen Druckknopf 109 und die dazugehörigen Aussparungen 110. Mittels dieser Verbindungsvorrichtung kann die Vorrichtung 100 ringförmig um eine Gliedmaße, vorzugsweise einen Arm des Patienten befestigt werden.

In die expandierbare Kammer 102 mündet eine Fluidzuführeinrichtung 103, durch die bei Verwendung der Vorrichtung 100 ein Fluid in die Kammer einführbar ist.

Eine Fördereinheit 108 fördert Fluid über die Fluidzuführeinrichtung 103 in die Kammer 102. Die Fördereinheit 108 kann beispielsweise eine Pumpe sein.

Die Steuerung 104 weist neben der Eingabevorrichtung 113 einen Speicher 106 auf, in dem mindestens ein Profil abgelegt ist, bei dessen Ausführung der Füllungsgrad der Kammer 102 reduzierbar ist. Die Eingabevorrichtung 113 kann Bedienelemente 111 und eine Anzeige 112 aufweisen. Mittels der Bedienelemente 111 kann ein Profil an- beziehungsweise auswählbar sein und die Anzeige 112 kann das Profil oder eine Mehrzahl von Profilen anzeigen. Die Anzeige des Profils kann als Graph, als Kennzahl oder als Beschreibung erfolgen und dementsprechend in dem Speicher gespeichert sein. Die Anzeige 112 kann die verbleibende Zeit, bis der Druck in der expandierbaren Kammer 102 abgebaut oder eine vorgegebene Zeit abgelaufen ist, anzeigen. Die Bedienelemente 111 und/oder die Anzeige 112 können in der Steuerung jeder beschriebenen Ausführungsform vorgesehen sein.

Das Ventil 105 zum Ablassen des Fluids aus der Kammer 102 kann beispielsweise eine Pumpe sein. Die Fördereinheit 108 und die Steuerung 104 können derart zusammenwirken, dass bei Verwendung der erfindungsgemäßen Vorrichtung 100 zunächst der Füllungsgrad in der Kammer 102 erhöht wird, wodurch sich die Kammer 102 ausdehnt und einen Druck auf die Einstichstelle ausübt. Anschließend kann die Steuerung 104 in Zusammenwirkung mit dem Ventil 105 gemäß einem vorbestimmten Profil den Füllungsgrad der Kammer 102 und demnach den Druck auf die Einstichstelle reduzieren.

Fig.1 b zeigt die erfindungsgemäße Vorrichtung 100 in aktivem Zustand. Der bandförmige Grundkörper 101 kann aus Kunststoff bestehen und die Kammer 102 kann aus zwei miteinander verbundenen Kunststoff-Schichten 107 gebildet sein. Durch die Fluidzuführeinrichtung 103 ist das sich in der Kammer 102 befindende Fluid mittels der Steuerung 104 und einem durch die Steuerung 104 ansteuerbaren Ventil 105, wieder ablassbar.

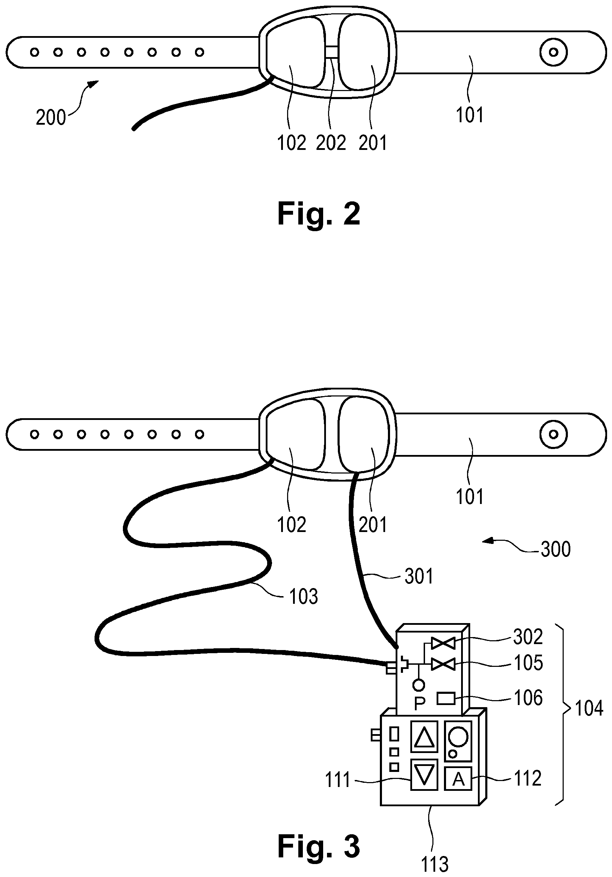

In Fig. 2 ist eine weitere Ausführungsform der erfindungsgemäßen Vorrichtung 200 dargestellt, die eine erste expandierbare Kammer 102 und eine zweite Kammer 201 aufweist. Die Vorrichtung 200 kann alle Merkmale der Vorrichtung 100, wie zu Figur 1 beschrieben, aufweisen. Die beiden Kammern 102 und 201 können über einen Kanal 202 verbunden sein. Über diesen Kanal kann Fluid gleichzeitig in beide Kammern einführbar und/oder ablassbar sein. Bei der Verwendung der Vorrichtung 200 gemäß dieser weiteren Ausführungsform kann die Blutung an zwei Einstichstellen gleichzeitig gestillt werden.

In Fig.3 ist eine weitere Ausführungsform der erfindungsgemäßen Vorrichtung 300 dargestellt. Die Vorrichtung 300 weist eine erste expandierbare Kammer 102 und eine zweite expandierbare Kammer 201 auf. Die Vorrichtung 300 kann alle Merkmale der Vorrichtung 200, wie zu Fig. 2 beschrieben, aufweisen. Allerdings sind in dieser Ausführungsform die Kammern 102 und 201 voneinander isoliert. Die Steuerung 104 der Vorrichtung 300, wie in Fig. 3 gezeigt, kann Fluid in die zweite Kammer 201 gesteuert über eine weitere Fluidzuführeinrichtung 301 zuführen und über ein weiteres Ventil 302 ablassen. Die Fluidzuführeinrichtung 301 bzw. das Ventil 302 können dieselben sein, über die das Fluid aus der ersten Kammer zuführ- bzw. ablassbar ist.

Bei der Verwendung der Vorrichtung 300 gemäß dieser weiteren Ausführungsform kann der Druck in der ersten Kammer 102 mit einem anderen Profil abgelassen werden als aus der zweiten Kammer 301.

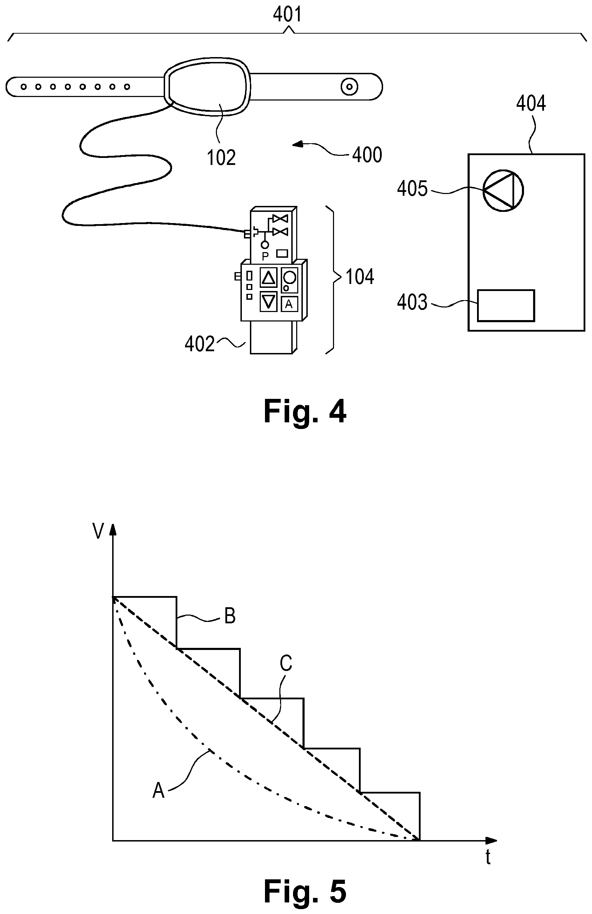

In Fig. 4 ist eine erfindungsgemäße medizintechnische Behandlungseinheit 401 dargestellt. Das System weist die Vorrichtung 400 gemäß einer der beschriebenen Ausführungsformen und eine extrakorporale Blutbehandlungsvorrichtung 404, hier beispielhaft ein Hämodialysegerät, auf. Die Steuerung 104 der Vorrichtung 400 weist eine erste Datenübertragungseinrichtung 402 auf und die Blutbehandlungsvorrichtung 404 weist eine zweite Datenüberragungseinrichtung 403 auf. Mittels der Datenübertragungseinrichtungen 402 und 403 können Daten von dem Blutbehandlungsvorrichtung 404 an die Vorrichtung 400 übertragen werden. Die übertragenen Daten können von der Steuerung 104 zum Anwählen oder Auswählen eines Profils verwendet werden. Das Füllen der Kammer 102 kann auch mittels einer Fördereinrichtung 405, die sich in der Blutbehandlungsvorrichtung 404 befindet, erfolgen.

In Fig. 5 sind beispielhaft drei Profile gezeigt, wie sie im Speicher 106 der Steuerung 104 gespeichert sein können. Die Profile zeigen die Reduktion des Füllungsgrades der Kammer 102 über die Zeit, wobei der Profilverlauf exponentiell (A), stufenförmig (B) oder linear (C) sein kann.

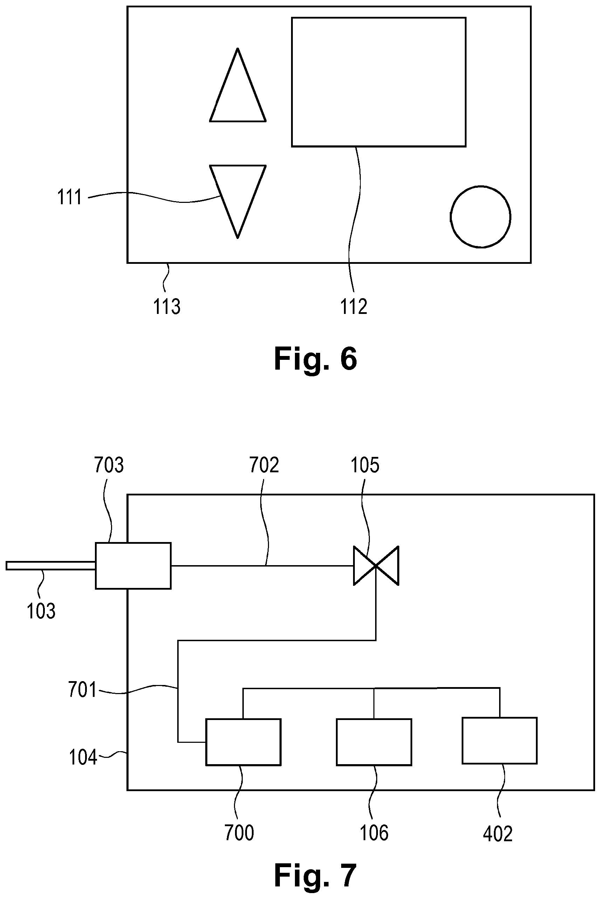

In Fig. 6 ist eine Ausführungsform der Steuerung 104 dargestellt. Die Steuerung 104 weist eine Anzeige 112 und Bedienelemente 111 auf. Mittels der Bedienelemente 111 kann ein Profil ausgewählt werden. Über die Bedienelemente 111 kann auch ein bestimmter Füllungsgrad der Kammer 102 einstellbar sein, indem das Ventil 105, gegebenenfalls zusammen mit der Fördereinheit 108, zum Ablassen und/ oder Einführen von Fluid ansteuerbar ist. Der Füllungsgrad der Kammer 102 kann mit einem Sensor gemessen werden.

In Fig. 7 ist eine Ausführungsform der Steuerung 104 mit einzelnen Bauteilen dargestellt. Die Steuerung 104 weist eine Datenübertragungseinrichtung 402, einen Prozessor 700, einen Speicher 106 und ein Ventil 105 auf. Eine Kommunikationsleitung 701 dient der Datenübertragung. Das Ventil 105 kann in einem Fluidleitungssystem 702 angeordnet sein, wobei das Fluidleitungssystem 702 geeignet ist, mit der Fluidzuführeinrichtung 103 verbunden zu werden. Die Steuerung 104 kann einen Zugang 703 zum Verbinden der Fluidzuführeinrichtung 103 mit der Steuerung 104 aufweisen. Bei der Verwendung einer Vorrichtung mit mehr als einer Kammer und mehreren Fluidzuführeinrichtungen können mehrere Zugänge zu dem Fluidleitungssystem vorgesehen sein.

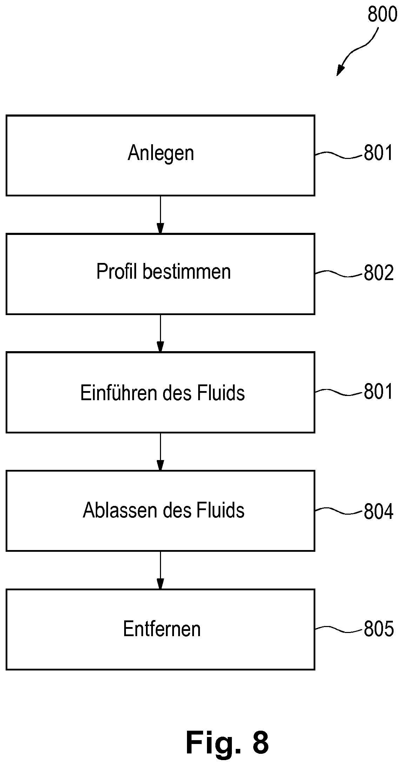

Fig. 8 zeigt ein Verfahren 800 zum Stillen einer Blutung an einer Einstichstelle an einem Gefäß eines Patienten. Das Verfahren umfasst die Schritte: Anbringen 801 der Vorrichtung mit der Kammer über der Einstichstelle, wobei die Vorrichtung um eine Gliedmaße, vorzugsweise den Arm des Patienten, gelegt wird, Bestimmen 802 eines Profils, gemäß dem der Druck in der Kammer abgebaut werden soll, Einführen eines Fluids 803 in die Kammer bis zu einem vorbestimmten Füllungsgrad, Ablassen 804 des Fluids gemäß des Profils. Entfernen 805 der Vorrichtung von der Gliedmaße, vorzugsweise von dem Arm des Patienten. Der Schritt des Einführens des Fluids 803 kann vor dem Schritt des Anbringens 801 oder des Bestimmens 802 erfolgen. Dies hat den Vorteil, dass es unabhängig vom Patienten erfolgen kann. Der Füllungsgrad kann dann beispielsweise durch die Verschlussvorrichtung eingestellt werden. Der Schritt des Bestimmens 802 des Profils kann den Schritt der Datenübertragung 805 von einer extrakorporalen Blutbehandlungsvorrichtung umfassen, wobei Daten die bei dem Schritt der Datenübertragung 805 übertragen werden, zur Bestimmung 802 des Profils verwendet werden können. Das Verfahren 800 kann mit jeder erfindungsgemäßen Vorrichtung durchgeführt werden.

### Bezugszeichenliste

- 100: Vorrichtung
- 101: bandförmiger Grundkörper
- 102: erste expandierbare Kammer
- 103: Fluidzuführeinrichtung
- 104: Steuerung
- 105: Ventil
- 106: Speicher
- 107: Kunststoff-Schicht
- 108: Fördereinheit
- 109: Druckknopf
- 110: Aussparungen
- 111: Bedienelemente
- 112: Anzeige
- 113: Eingabevorrichtung
- 200: alternative Ausführungsform der Erfindung
- 201: zweite expandierbare Kammer
- 202: Kanal
- 300: alternative Ausführungsform der Erfindung
- 301: zweite Fluidzuführeinrichtung
- 302: zweites Ventil
- 400: alternative Ausführungsform der Erfindung
- 401: medizintechnische Behandlungseinheit
- 402: erste Datenübertragungseinrichtung
- 403: zweite Datenübertragungseinrichtung
- 404: Blutbehandlungsvorrichtung
- 405: Fördereinrichtung
- 700: Prozessor
- 701: Kommunikationsleitung
- 702: Fluidleitungssystem
- 703: Zugang zum Verbinden der Fluidzufuhreinrichtung
- 800: Verfahren zum Stillen von Blutungen
- 801: Anlegen
- 802: Profil bestimmen
- 803: Einführen des Fluids
- 804: Ablassen des Fluids
- 805: Entfernen
- A: Profil: exponentieller Verlauf
- B: Profil: stufenförmiger Verlauf
- C: Profil: linearer Verlauf

## Patentansprüche

1. Medizinische Behandlungseinheit die eine Vorrichtung (100) zum Stillen von Blutungen an mindestens einer Einstichstelle eines Gefäßes eines Patienten, der einer extrakorporalen Behandlung, insbesondere einer Dialyse, unterzogen wurde, aufweist, wobei die Vorrichtung (100):
- einen bandförmigen Grundkörper (101) mit Verschlussmittel (109);
- wenigstens eine erste expandierbare Kammer (102) an dem bandförmigen Grundkörper (101);
- eine Fluidzuführeinrichtung (103), die derart angeordnet ist, dass das Fluid durch diese in die wenigstens erste expandierbare Kammer (102) einführbar und/oder aus dieser ablassbar ist;
- eine Fördereinheit (108) zum Einführen eines Fluids in die wenigstens erste Kammer (102);
- ein Ventil (105) zum Ablassen des Fluids aus der wenigstens ersten Kammer (102); und
- eine Steuerung (104) zum Ansteuern der Fördereinheit (108) und/oder des Ventils (105) aufweist, wobei die Steuerung (104) einen Speicher (106) aufweist, in dem wenigstens ein Profil zum gesteuerten Einführen in die und/oder Ablassen des Fluids aus der wenigstens ersten Kammer (102) gespeichert ist,
wobei die Steuerung (104) eine Datenübertragungseinrichtung (402) und einen Prozessor (700) aufweist, wobei der Prozessor geeignet ist, auf der Grundlage von mittels der Datenübertragungseinrichtung (402) übertragenen Daten das Profil zu bestimmen, wobei die Behandlungseinheit eine extrakorporale Blutbehandlungsvorrichtung, insbesondere eine Hämodialysevorrichtunc aufweist, wobei die extrakorporale Blutbehandlungsvorrichtung eingerichtet ist, mit der Datenübertragungseinrichtung (402) zu kommunizieren.

2. Behandlungseinheit nach Anspruch 1, **dadurch gekennzeichnet, dass**
der Grundkörper (101) der Vorrichtung (100) wenigstens in einem Teil des Bereichs der ersten Kammer (102) auf einer Außenseite steifer ausgebildet ist als auf einer Innenseite, wenn der bandförmige Grundkörper (101) ringförmig gebogen ist.

3. Behandlungseinheit nach einem der vorhergehenden Ansprüche, **dadurch gekennzeichnet, dass**
die wenigstens erste Kammer (102) der Vorrichtung (100) zumindest teilweise transparent ist.

4. Behandlungseinheit nach einem der vorhergehenden Ansprüche, **dadurch gekennzeichnet, dass**
die wenigstens erste Kammer (102) der Vorrichtung (100) zumindest teilweise flexibel, vorzugsweise elastisch, ist.

5. Behandlungseinheit nach einem der vorhergehenden Ansprüche, wobei die Abmessungen der Kammer (102) der Vorrichtung (100) in Längsrichtung geringer ist als die Abmessungen des Grundkörpers (101), vorzugsweise weniger als die Hälfte der Abmessungen des Grundköpers (101) beträgt.

6. Behandlungseinheit nach einem der vorhergehenden Ansprüche, **dadurch gekennzeichnet, dass**
die Vorrichtung (100) eine Eingabevorrichtung (113) enthält, mit der das wenigstens eine Profil anwählbar ist.

7. Behandlungseinheit nach einem der vorhergehenden Ansprüche, **dadurch gekennzeichnet, dass**
die Vorrichtung (100) einen Sensor zur Messung des Füllungsgrads der wenigstens ersten Kammer enthält.

8. Behandlungseinheit nach Anspruch 7, **dadurch gekennzeichnet, dass**
die Fördereinheit (108) und/oder das Ventil (105) auf der Basis des gemessenen Füllungsgrads ansteuerbar sind.

9. Behandlungseinheit nach einem der vorhergehenden Ansprüche, **dadurch gekennzeichnet, dass**
die Fördereinheit (108) einen Druck von 6666 Pascal (50 mmHg) bis 19999 Pascal (150 mmHg) erzeugen kann.

10. Behandlungseinheit nach einem der vorhergehenden Ansprüche, **dadurch gekennzeichnet, dass**
eine zweite Kammer (201) in einem Abstand von der ersten Kammer (102) an dem Grundkörper (101) vorhanden ist, wobei die erste und die zweite Kammer (101; 201) über einen fluiddurchlässigen Kanal (202) verbunden sind und eine der Kammern (101; 201) mindestens eine Zuführ- bzw. Ablassstelle für ein Fluid aufweist.

11. Behandlungseinheit nach einem der Ansprüche 1 bis 9 **dadurch gekennzeichnet, dass**
eine zweite Kammer (201) in einem Abstand von der ersten Kammer (102) an dem Grundkörper (101) vorhanden ist, wobei
die erste und die zweite Kammer (101; 201) isoliert voneinander angeordnet sind und jeweils mindestens eine Zuführ- bzw. Ablassstelle für ein Fluid aufweisen.

12. Behandlungseinheit nach einem der vorhergehenden Ansprüche, **dadurch gekennzeichnet, dass**
es sich bei der Datenübertragungseinrichtung (402) um eine Schnittstelle der Gruppe bestehend aus einer drahtlosen Kommunikationsschnittstelle und einer drahtgebundene Kommunikationsschnittstelle handelt.

13. Behandlungseinheit nach Anspruch 12, **dadurch gekennzeichnet, dass** es sich bei den Daten um Daten der Gruppe bestehend aus Patientenidentifikation, Blutviskosität, Hämatokrit, Fistel- bzw. Shuntdruck, profilspezifische Kennzahl, Dialysebehandlungsparameter, ACT ("activated clotting time") oder Antikoagulationsregimeparameter handelt.

14. Behandlungseinheit nach einem der Ansprüche 1 bis 13, **dadurch gekennzeichnet, dass** die Steuerung (104) zumindest einen Sensor umfasst, der den Füllungsgrad der wenigstens ersten expandierbaren Kammer (102) misst.

## Claims

1. Medical treatment unit having a device (100) for stopping bleeding at at least one puncture site of a vessel of a patient who has undergone an extracorporeal treatment, in particular dialysis, wherein the device (100) has:
- a band-shaped main body (101) with closure means (109) ;
- at least one first expandable chamber (102) on the band-shaped main body (101);
- a fluid feed device (103), which is arranged in such a way that the fluid can be introduced through it into the at least first expandable chamber (102) and/or can be discharged through it;
- a delivery unit (108) for introducing a fluid into the at least first chamber (102);
- a valve (105) for discharging the fluid from the at least first chamber (102), and
- a controller (104) for controlling the delivery unit (108) and/or the valve (105),
wherein the controller (104) has a memory (106), in which at least one profile is stored for controlled introduction and/or discharge of the fluid into/out of the at least first chamber (102),
wherein the controller (104) has a data transmission device (402) and a processor (700), wherein the processor is suitable for determining the profile on the basis of data transmitted by means of the data transmission device (402),
wherein the treatment unit has an extracorporeal blood treatment device, in particular a haemodialysis device, wherein the extracorporeal treatment device is equipped to communicate with the data transmission device (402).

2. Treatment unit according to Claim 1, **characterized in that** the main body (101) of the device (100), at least in one part of the region of the first chamber (102), is designed to be stiffer on an outer face than on an inner face, when the band-shaped main body (101) is bent in a ring shape.

3. Treatment unit according to one of the preceding claims, **characterized in that** the at least first chamber (102) of the device (100) is at least partially transparent.

4. Treatment unit according to one of the preceding claims, **characterized in that** the at least first chamber (102) of the device (100) is at least partially flexible, preferably elastic.

5. Treatment unit according to one of the preceding claims, wherein the dimensions of the chamber (102) of the device (100) in the longitudinal direction are smaller than the dimensions of the main body (101), preferably smaller than one-half of the dimensions of the main body (101).

6. Treatment unit according to one of the preceding claims, **characterized in that** the device (100) includes an input device (113), with which the at least one profile can be selected.

7. Treatment unit according to one of the preceding claims, **characterized in that** the device (100) includes a sensor for measuring the degree of filling of the at least first chamber.

8. Treatment unit according to Claim 7, **characterized in that** the delivery unit (108) and/or the valve (105) can be controlled on the basis of the measured degree of filling.

9. Treatment unit according to one of the preceding claims, **characterized in that** the delivery unit (108) can generate a pressure of 6,666 Pascal (50 mmHg) to 19,999 Pascal (150 mmHg).

10. Treatment unit according to one of the preceding claims, **characterized in that** a second chamber (201) is present on the main body (101) at a distance from the first chamber (102), wherein the first and second chambers (101; 201) are connected via a fluid-permeable channel (202), and one of the chambers (101; 201) has at least one feed location and/or discharge location for a fluid.

11. Treatment unit according to one of Claims 1 to 9, **characterized in that** a second chamber (201) is present on the main body (101) at a distance from the first chamber (102), wherein the first and second chambers (101; 201) are arranged isolated from each other, and each of them has at least one feed location and/or discharge location for a fluid.

12. Treatment unit according to one of the preceding claims, **characterized in that** the data transmission device (402) is an interface from the group consisting of a wireless communication interface and a wired communication interface.

13. Treatment unit according to Claim 12, **characterized in that** the data are data from the group consisting of patient identification, blood viscosity, haematocrit, fistula pressure and/or shunt pressure, profile-specific code number, dialysis treatment parameter, ACT (activated clotting time) or anticoagulation regimen parameter.

14. Treatment unit according to one of Claims 1 to 13, **characterized in that** the controller (104) comprises at least one sensor which measures the degree of filling of the at least first expandable chamber (102).

## Revendications

1. Unité de traitement médical qui comporte un dispositif (100) destiné à arrêter des hémorragies au niveau d'au moins un site de ponction d'un vaisseau d'un patient ayant subi un traitement extracorporel, en particulier une dialyse, le dispositif (100) comportant :
- un corps de base (101) en forme de bande pourvu d'un moyen de fermeture (109) ;
- au moins une première chambre expansible (102) sur le corps de base (101) en forme de bande ;
- un moyen d'alimentation en fluide (103) qui est disposé de telle sorte que le fluide puisse être introduit par le biais de celui-ci dans l'au moins une première chambre expansible (102) et/ou être évacué de celle-ci ;
- une unité d'acheminement (108) destinée à introduire un fluide dans l'au moins une première chambre (102) ;
- une soupape (105) destinée à évacuer le fluide de l'au moins une première chambre (102) ; et
- une commande (104) destinée à commander l'unité d'acheminement (108) et/ou la soupape (105),
la commande (104) comportant une mémoire (106) dans laquelle au moins un profil d'introduction commandée du fluide dans l'au moins une première chambre (102) et/ou d'évacuation commandée du fluide de l'au moins une première chambre (102) est mémorisé,
la commande (104) comportant un moyen de transmission de données (402) et un processeur (700), le processeur étant adapté pour déterminer le profil sur la base de données transmises à l'aide du moyen de transmission de données (402),
l'unité de traitement comportant un dispositif de traitement de sang extracorporel, en particulier un dispositif d'hémodialyse,
le dispositif de traitement de sang extracorporel étant adapté pour communiquer avec le moyen de transmission de données (402).

2. Unité de traitement selon la revendication 1, **caractérisée en ce que**
le corps de base (101) du dispositif (100) est conçu pour être plus rigide d'un côté extérieur que d'un côté intérieur, au moins dans une partie de la région de la première chambre (102), lorsque le corps de base (101) en forme de bande est plié annulairement.

3. Unité de traitement selon l'une des revendications précédentes, **caractérisée en ce que**
l'au moins une première chambre (102) du dispositif (100) est au moins partiellement transparente.

4. Unité de traitement selon l'une des revendications précédentes, **caractérisée en ce que**
l'au moins une première chambre (102) du dispositif (100) est au moins partiellement flexible, de préférence élastique.

5. Unité de traitement selon l'une des revendications précédentes, les dimensions de la chambre (102) du dispositif (100) dans la direction longitudinale étant inférieures aux dimensions du corps de base (101), de préférence inférieures à la moitié des dimensions du corps de base (101).

6. Unité de traitement selon l'une des revendications précédentes, **caractérisée en ce que**
le dispositif (100) contient un dispositif d'entrée (113) permettant de sélectionner l'au moins un profil.

7. Unité de traitement selon l'une des revendications précédentes, **caractérisée en ce que**
le dispositif (100) contient un capteur destiné à mesurer le niveau de remplissage de l'au moins une première chambre.

8. Unité de traitement selon la revendication 7, **caractérisée en ce que**
l'unité d'acheminement (108) et/ou la soupape (105) peuvent être commandées sur la base du niveau de remplissage mesuré.

9. Unité de traitement selon l'une des revendications précédentes, **caractérisée en ce que**
l'unité d'acheminement (108) peut générer une pression de 6666 Pascal (50 mmHg) à 19999 Pascal (150 mmHg).

10. Unité de traitement selon l'une des revendications précédentes, **caractérisée en ce que**
une deuxième chambre (201) est prévue sur le corps de base (101) à distance de la première chambre (102), la première et la deuxième chambre (101 ; 201) étant reliées par un canal (202) perméable au fluide et l'une des chambres (101 ; 201) comportant au moins un site d'alimentation ou d'évacuation d'un fluide.

11. Unité de traitement selon l'une des revendications 1 à 9, **caractérisée en ce que**
une deuxième chambre (201) est prévue sur le corps de base (101) à distance de la première chambre (102),
la première et la deuxième chambre (101 ; 201) étant disposées de manière isolée l'une de l'autre et comportant chacune au moins un site d'alimentation ou d'évacuation d'un fluide.

12. Unité de traitement selon l'une des revendications précédentes, **caractérisée en ce que**
le moyen de transmission de données (402) est une interface du groupe comprenant une interface de communication sans fil et une interface de communication filaire.

13. Unité de traitement selon la revendication 12, **caractérisée en ce que**
les données sont des données du groupe comprenant l'identification du patient, la viscosité du sang, l'hématocrite, la pression de fistule ou de shunt, le numéro d'identification spécifique au profil, les paramètres du traitement de dialyse, l'ACT ('activated clotting time' = 'temps de coagulation activé') ou les paramètres du régime d'anti-coagulation.

14. Unité de traitement selon l'une des revendications 1 à 13, **caractérisée en ce que**
la commande (104) comprend au moins un capteur qui mesure le niveau de remplissage de l'au moins une première chambre expansible (102).
